(19) Europäisches Patentamt
European Patent Office
Office européen des brevets

(11) Publication number: **0 076 217**
**A2**

# (12) EUROPEAN PATENT APPLICATION

(21) Application number: 82401770.1

(22) Date of filing: 29.09.82

(51) Int. Cl.³: C 07 C 76/00
C 07 C 77/00

(30) Priority: 30.09.81 US 307201

(43) Date of publication of application:
06.04.83 Bulletin 83/14

(84) Designated Contracting States:
AT BE DE FR GB IT NL SE

(71) Applicant: UNION CARBIDE CORPORATION
Old Ridgebury Road
Danbury Connecticut 06817(US)

(72) Inventor: Doumaux, Arthur, Jr.
Roy, 1401 Wilkie Drive
Charleston West Virginia 25314(US)

(72) Inventor: Downey, James Michael
2360 Harrison Avenue
St. Albans West Virginia 25177(US)

(72) Inventor: Henry, Joseph Peter
453 Forest Circle
South Charleston West Virginia 25303(US)

(72) Inventor: Hurt, John Marion
2613 East Parkview Drive
St. Albans West Virginia 25177(US)

(74) Representative: Rinuy, Guy et al,
Cabinet Rinuy et Santarelli 14, Avenue de la Grande Armée
F-75017 Paris(FR)

## (54) Preparation of nitrite esters in vapor phase from nitrogen oxide and alcohol.

(57) The invention deals with the preparation of nitrite esters in vapor phase from nitrogen oxide, alcohol and an inert diluent.

The reaction, which occurs between 50 and 140°C at or above atmospheric pressure, takes place into a first reaction zone optimizing the reaction $N_2O_3 + ROH \rightarrow RONO + HONO$ and a second reaction zone optimizing the reaction $ROH + HONO \rightarrow RONO + H_2O$.

The formation of acid by-products is minimized as compared to a unitary reaction zone.

EP 0 076 217 A2

0076217

# PREPARATION OF NITRITE ESTERS IN VAPOR PHASE FROM NITROGEN OXIDE AND ALCOHOL

## FIELD OF THE INVENTION

This invention relates to a process for the preparation of nitrite esters. More particularly, the present invention relates to the preparation of nitrite esters of aliphatic alcohols in a vapor phase synthesis from the reaction of an aliphatic alcohol and a nitrogen oxide composition under relatively mild operating conditions.

## BACKGROUND OF THE INVENTION

Nitrite esters, i.e., esters of nitrous acid, are generally colorless or pale yellow liquids which have found use in areas such as additives to motor fuels, stabilizers for vinyl compounds, as spasmolytic agents, as reagents for diazotization and as reagents for chemical synthesis.

The classical method for preparing nitrite esters involves the liquid phase reaction of sodium nitrite and sulfuric acid with a desired alcohol. The reaction is normally carried out at ice temperatures, due to the extremely exothermic nature of the reaction, to form nitrite esters as follows :

$$2NaNO_2 + H_2SO_4 + 2ROH \longrightarrow 2RONO + Na_2SO_4 + 2H_2O$$

The nitrite ester formed is insoluble in water (less than about 1 percent in water or water in the nitrite ester) so that the nitrite ester may be separated from the reaction products.

The production of nitrite esters in the liquid phase is disclosed in U.S. Patent No. 2,166,698 wherein nitrite esters are produced by reacting an appreciably water soluble open-chain saturated aliphatic compound containing a plurality of esterifiable carbinol groups with nitrous acid in an aqueous medium and removing a nitrite ester from the reaction system substantially as soon as it is formed therein. The nitrite esters formed therein react rapidly with alcohol by ester interchange, e.g., ethyl alcohol, to form

an alkyl nitrite, e.g., ethyl nitrite.

U.S. Patent No. 2,739,166 describes producing alkyl nitrites in a liquid phase process by bubbling nitrogen dioxide gas into a cooled liquid monohydric aliphatic alcohol but nitric acid is formed as a by-product.

In British Patent Specification No. 586,022, a liquid phase process for preparing esters of nitrous acid by continuously removing the formed ester by employing alcohol in molar excess over dinitrogen trioxide at temperatures below the boiling point of the alcohol and simultaneously distilling off the ester formed. In addition, the reference acknowledges that the vapor phase decomposition of alcohols with nitrogen dioxide-nitrogen monoxide mixtures at temperatures between 100 and 420° is known.

Japanese Application No. 53-8268/78 describes the preparation of nitrite esters by a conventional liquid phase process as part of the continuous production of oxalic acid diester using nitrite ester as a starting material. The nitrous acid ester in the process is formed by employing a common gas-liquid contacting apparatus to react nitrogen oxides with an alcohol at a temperature lower than the boiling point of the alcohol.

The aforementioned processes are to be distinguished from a vapor phase process in that in liquid phase processes the separation of the nitrite ester product is difficult and oxidation of alcohol in the liquid phase during the manufacture or separation can occur to form unwanted by-products. In addition, the separation of the highly flammable and toxic nitrite ester from the liquid phase can prove to be a major safety and health problem.

A vapor phase process is disclosed in U.S. Patent No. 2,831,822. This patent discloses a process for the preparation of nitrite esters which comprises reacting a vaporized alcohol with from 0.4 and 0.6 mole of nitrogen dioxide and 0.4 and 2.0 moles of nitric oxide per mole of alcohol in the presence of from 2 to 25 moles of diluent which may be water, nitrogen, or carbon dioxide, at a temperature between 100°C and 420°C with a contact time of 1-10 seconds in a reaction vessel comprising a simple tube or cylindrical

vessel.

Table 1 of U.S. Patent No. 2,831,882 describes 4 examples wherein the molar ratio of NO to $NO_2$ is greater than one but wherein the molar ratio of alcohol to combined NO and $NO_2$ needed to react with all the $N_2O_3$ possible, is less than 1. In each case, in order to achieve a relatively high conversion, above 80%, it was necessary to employ temperatures in excess of about 130°C. In addition to the increased rate of decomposition of nitrite ester (product) at these temperatures, the reference creates additional problems by requiring the employment in each example of a significant amount of water. The use of water in the process results in the formation of nitric acid at least some of which will be present in the ester product.

Table II of U.S. Patent No. 2,831,882 describes examples which employ various molar ratios of nitric acid, nitrogen dioxide, nitric oxide and nitrogen dioxide or nitric acid to alcohol (n-butanol). In each example the molar ratio of alcohol to total nitrogen oxides is less than one. Further, in each example, a temperature in excess of 170°C was required to provide a conversion to nitrite ester product greater than 70 percent. In addition, the patent, at column 3, lines 55 to 64, states that :

> "When nitrogen dioxide is reacted with the alcohol in the presence of water at temperatures below 250°C, equimolar proportions of the nitrite ester and nitric acid are formed. By increasing the temperature of the reaction to 350°C, the formation of nitric acid was almost eliminated, and the conversion to nitrite ester increased. These results are consistent with the previously mentioned mechanism of reaction, since a higher temperature increases the decomposition of both nitric acid and nitrogen dioxide".

Thus, not only does the process require relatively high temperatures but also results in the formation of nitric acid which may be decomposed at higher temperatures. These results are, in part, the net effect of employing a process which fails to accomodate the unique chemistry of the reaction that results in the formation of the ester of nitrous acid.

Example 1 of U.S. Patent No. 2,831,882 prepares the nitrite of isopropyl alcohol. This example in U.S. Patent 2,831,882 provides a molar ratio of NO to $NO_2$ of less than one and an isopropanol to a combined NO and $NO_2$ molar ratio of greater than one. The process is operated at a pressure of $6.2 \times 10^5$ Pa with only a 58 percent conversion to product (the reference reports a yield of 89% based, presumably, on nitrite ester converted from the alcohol consumed which in reality is a conversion of about 39 percent based on the nitrite oxide and nitrogen dioxide available). In addition, the process results in the incomplete reaction of the nitrogen dioxide. The unreacted nitrogen dioxide may be quite delitereous to any further process in which the nitrite ester is employed.

U.S. Patent No. 4,229,591 uses the preparation of nitrite esters as an intermediate step in a process for preparing a diester of oxalic acid. The patent discloses, at Column 2, lines 21-35, that :

> "The nitrogen compound used in the present process need not necessarily be in the form of an ester of nitrous acid, and a compound which forms an ester of nitrous acid in the reaction system may also be used. It may also be advantageous to use an alcohol along with a nitrogen compound selected from the group consisting of nitrogen monoxide, nitrogen dioxide, dinitrogen trioxide and dinitrogen tetraoxide, and hydrates of a nitrogen oxide instead of an ester of nitrous acid by introducing a gas containing molecular oxygen into the system in cases where nitrogen monoxide is used. As the hydrates of a nitrogen oxide may effectively be used nitric acid, nitrous acid and the like. An alcohol to be used in such cases is selected from alcohols which constitute esters of nitrous acid as mentioned hereinbelow".

To overcome the problems associated with the known processes for the preparation of nitrite esters a process must be found that may be run in the vapor phase at relatively low temperatures and pressures while minimizing the formation of acid by-products, e.g. nitric acid and nitrate esters.

The aforementioned processes fail to appreciate the need to provide a specially designed process configuration for the vapor phase process for making the esters of nitrous acid. The instant process provides such a process configuration.

## SUMMARY OF THE INVENTION

This invention relates to the preparation of esters of nitrous acid in the vapor state in two reaction zones, whereby the formation of the esters of nitrous acid is effected, while the formation of acid by-products is minimized with increasing rate to products by employing two reaction zones. The formation of by-products is minimized as compared to employing only a unitary reaction zone. Preferably, the preparation is carried out with : (i) a molar amount of a nitrogen oxide composition containing a nitric oxide to nitrogen dioxide molar ratio of greater than 1, and (ii) a molar amount of an aliphatic alcohol wherein the molar ratio of aliphatic alcohol to the molar amount of the nitrogen oxide composition (theoretical amount of $N_2O_3$ formed) is greater than one. The preparation of the ester of nitrous acid is carried out in two reaction zones, hereinafter described, in the presence of an inert gaseous diluent for said reaction, at a temperature and pressure sufficient to form said esters of nitrous acid, preferably at a temperature in each reaction zone of at least about 10°C to about 300°C, at a pressure in each reaction zone at atmospheric or superatmospheric pressure for a period of time sufficient to form the ester of nitrous acid.

## DESCRIPTION OF FIGURES

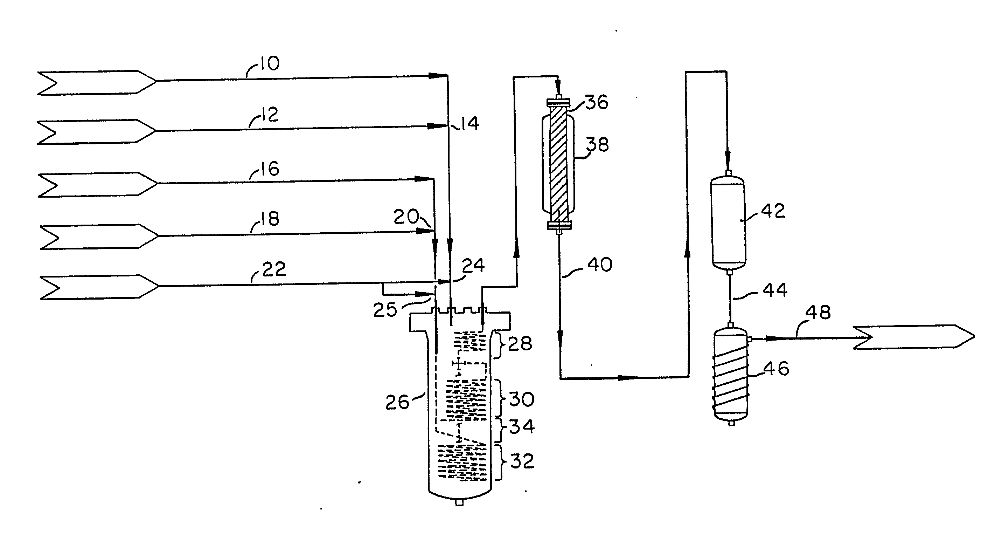

Figure 1 depicts an apparatus employing a primary and secondary reaction zones for the preparation of esters of nitrous acid according to the invention.

## DETAILED DESCRIPTION

The instant invention relates to a novel process for the preparation of nitrite esters, and is particularly well suited for the manufacture of methyl and/or ethyl nitrite. The process involved

in the preparation of the esters of nitrous acid may be understood more fully by reference to the following equations.

$$(1)\quad 2NO + O_2 \rightleftharpoons 2NO_2$$

$$(2)\quad NO_2 + NO \rightleftharpoons N_2O_3$$

$$(3)\quad 2ROH + N_2O_3 \rightleftharpoons 2RONO + H_2O$$

$$(4)\quad ROH + N_2O_3 \longrightarrow RONO + HONO$$

$$(5)\quad ROH + HONO \rightleftharpoons RONO + H_2O$$

$$(6)\quad 2NO_2 \rightleftharpoons N_2O_4$$

$$(7)\quad ROH + N_2O_4 \longrightarrow RONO + HNO_3$$

wherein R is a monovalent hydrocarbon derived from an aliphatic alcohol.

Since the goal of this, as well as any, process is to optimize the production of products, e.g. the esters of nitrous acid, while minimizing, preferably essentially eliminating, the formation of by-products, and in the instant process, particularly acid products. The reactions characterized by equations (1), (2), (3) are integrated in reaction (4) which supplies the nitrous acid for reaction (5) and accordingly reactions (4) and (5) are preferred. The reaction sequence of equations (1)-(5) is preferred while the reactions characterized by equations (6) and (7) are to be minimized because of the formation of nitric acid. It has been discovered that reactions (4) and (5) are not optimized in the same manner and that if the overall process is to be optimized such that the rate of formation of RONO is increased while by-product formation is minimized that this difference in reactions (4) and (5) must be taken into consideration.

Copending U.S. Serial No. 239,761, incorporated by reference herein, discloses a novel process wherein the reaction sequence of equations (1)-(5) is enhanced by providing NO, $NO_2$ and ROH in specific molar ratios such that alkyl nitrite may be formed in high yield with a substantial decrease in the formation of acid by-products (reported as nitric acid). To achieve these results a

molar ratio of nitric oxide to nitrogen dioxide (formed from the reaction of nitric oxide and oxygen) is provided such that it is greater than one and the molar ratio of alcohol to the combined molar amount of nitric oxide and nitrogen dioxide (i.e., that amount which forms $N_2O_3$ according to equation (2)) is greater than one. The correlation of these two molar ratios provides the novel process disclosed therein.

Although the process of copending U.S. Serial No. 239,761 achieves dramatic improvements in the decrease of objectionable acid by-products, such as nitric acid, it has been discovered that the nature of the reaction for the formation of the esters of nitrous acid is not carried out advantageously in a single reaction zone owing to the different routes to the ester product, as exemplified by equations (4) and (5), and that a dramatic improvement in the decrease of acid by-products formed in the process may be achieved by employing two reaction zones. Owing to the different reaction schemes for forming the ester of nitrous acid it is necessary that the preparation be carried out in two reactions having different characteristics if an efficient process is to be achieved.

It has been discovered that the process for preparation of the esters of nitrous acid may be carried out employing two reaction zones with a first reaction zone (primary reaction zone) wherein the ester of nitrous acid is formed by optimizing the reaction of dinitrogen trioxide and aliphatic alcohol (equation (4) above) and a second reaction zone (secondary reaction zone) wherein the reaction of nitrous acid and aliphatic alcohol (equation (5) above) is optimized. The first reaction zone is characterized as being substantially free of radical quenching sites such that the first reaction zone optimizes the reaction characterized by equation (4). Accordingly, the first reaction zone is generally a tubular reactor through which the reactants (i.e., aliphatic alcohol and nitrogen oxide compound) and diluent are passed. The second reaction zone is a reaction zone characterized by having a relatively large surface area by way of being filled with packing material in the secondary

reaction zone such that the secondary reaction zone optimizes the reaction characterized by equation (5). The secondary reaction zone preferably is packed with a packing having a surface area that is sufficient to optimize the reaction of nitrous acid and alcohol to the product, i.e., ester of nitrous acid. Typical packing materials are glass and ceramic materials, although other packing materials and various shapes and sizes may be employed.

It has been discovered that by employing this process configuration that the overall process for preparing the esters of nitrous acid may be optimized as is indicated by the decrease in the formation of acid by-products. Such acid by-products must generally be removed from the product mixture prior to use of the product mixtures in subsequent processes, those increase the corrosiveness of the product mixture, and provide a means for promoting the formation of other unwanted by-products.

In carrying out the process the source of the reactants is not critical. Nitric oxide may be provided by the decomposition of nitric acid and/or nitrogen dioxide, or may be introduced from a source such as an ammonia oxidation unit. The process will generally be carried out by introducing nitric oxide and oxygen to form the required amounts of nitrogen dioxide (see equation (1)). The molar ratio of nitric oxide to nitrogen dioxide is preferably maintained above one, in this case by providing nitric oxide and oxygen at a molar ratio of greater than 4 to 1, such that the molar ratio of nitric oxide to the nitrogen dioxide is greater than 1. A gaseous medium having the desired ratio of nitric oxide to nitrogen dioxide may be obtained by use of higher oxides of nitrogen ($N_2O_3$, $N_2O_4$, $N_2O_5$, etc.) and to the extent that such higher oxides may be employed to provide a gaseous medium, with or without the addition of molecular oxygen, having a molar ratio of NO to $NO_2$ greater than 1, said higher oxides may be employed herein. In addition, compounds such as nitrous acid, which can decompose and react to provide a gaseous medium having a molar ratio of NO to $NO_2$ greater than one may be employed and are preferred.

As noted above, the process is preferably carried out by forming the desired molar ratio of NO to $NO_2$ by reacting molecular

oxygen and NO at a molar ratio of 4 to 1 or greater. The process may be carried out by mixing nitric oxide, oxygen, and aliphatic alcohol together at the desired molar ratios. Although such mixing may be undesirable because oxygen may oxidize some of the alcohol (methanol or ethanol) and result in the loss of valuable starting material, there are certain advantages to be obtained from introducing the nitrogen oxide composition, alcohol and oxygen in a single step. The reaction of a nitrogen oxide composition with oxygen is exothermic in nature and the heat generated by the reaction must be removed from the process unless it can be used. By mixing the nitrogen oxide composition, oxygen and alcohol together the heat of reaction of oxygen and the nitrogen oxide composition may be employed to vaporize alcohol introduced as a mist, i.e., finely dispersed liquid. Although this means of effecting the reaction may be preferred it may also be preferable in some instances to react the nitrogen compound and oxygen prior to addition of alcohol to minimize the oxidation of alcohol by oxygen.

The preferred esters of nitrous acid are esters derived aliphatic alcohols such as saturated monohydric aliphatic alcohols to such as those formed from a saturated monohydric open-chain aliphatic alcohol having 1 to 8 carbon atoms or an alicyclic alcohol having 1 to 8 carbon atoms. The most preferred esters of nitrous acid are those prepared from methanol and ethanol. As the alcohol component may be mentioned aliphatic alcohols such as methanol, ethanol, n-propanol, isopropanol, n-butanol; isobutanol, sec-butanol, tert-butanol, n-amyl alcohol, isoamyl alcohol, hexanol, octanol, etc., and alicyclic alcohols such as cyclohexanol, methylcyclohexanol, etc. These alcohols may contain therein a substituent such as an alkoxy group which does not inhibit the reaction.

The process is generally carried out in the presence of an inert gaseous diluent to moderate the reaction to preclude the formation of explosive mixtures and prevent the formation of excessive amounts of undesirable by-products. When carrying out the process the inert gaseous diluent is added either concurrently

with the nitrite oxide or with the molecular oxygen, or with both. Further, inert gaseous diluent may be added to carry and vaporize the alcohol. As the inert gaseous diluent, it is preferred to employ nitrogen, carbon dioxide or other inert gaseous compounds. The use of carbon dioxide provides higher heat capacity relative to nitrogen. Carbon monoxide may be present and used as a diluent although its concentration in the reaction system must be carefully controlled to prevent the formation of flammable mixtures. The inert gaseous diluent is employed in a sufficient amount to provide the aforementioned objectives. The inert diluent is generally employed in the process such that between about 1 and about 99 mole percent, preferably between about 30 and about 90 mole percent, and most preferably between about 30 and about 70 mole percent, is inert gaseous diluent. The exact amount of inert gaseous diluent will, in part, be determined by the selected ester of nitrous acid and the selected process parameters, e.g., temperature and pressure.

According to the invention, the process is carried out such that the temperature in the primary and secondary reaction zone is between about 10°C and about 300°C, preferably between about 50°C and about 140°C and most preferably between greater than 95°C and less than about 120°C. The lowest temperature at which the process is conducted is generally determined by the dew point of the aliphatic alcohol employed and the concentration of acid by-product to be formed. It has been found that at temperatures greater than 95°C that the formation of acid by-products is greatly diminished. This is especially true if the temperature is correlated to the ratio of alcohol and the theoretical amount of $N_2O_3$ (based on the molar amount of nitrogen compound employed).

The pressure in the primary and secondary reaction zones is not narrowly critical. Preferably, atmospheric or superatmospheric pressure is employed, more preferably between about atmospheric pressure (14.7 psia) and about $6.9 \times 10^5$ Pa absolute pressure (100 psia) and most preferably at between about $1.38 \times 10^5$ Pa and about $4.13 \times 10^5$ Pa absolute pressure (20 and 60 psia). Pressures less than atmospheric pressure (14.7 psia) may be employed, if desired.

The process is preferably carried out with reactants which are essentially anhydrous since the presence of water in the reactants fosters the formation of undesirable by-products which must be separated ultimately if the ester of nitrous acid is to be subsequently employed in further processes. It is preferred to carry out the process such that the amount of water provided by the reactants is minimized. It should be remembered that the reaction forms water and such formed water must be tolerated or removed by condensation or other means.

As stated, the molar ratio of nitric oxide to nitrogen dioxide is preferably greater than 1. Typically the molar ratio (NO to $NO_2$) will be from greater than 1 to about 10, preferably from greater than 1 to about 2 and most preferably from greater than 1 to about 1.5. The molar ratio of alcohol (methanol or ethanol) to the combined molar amount of nitric oxide and nitrogen dioxide is also - preferably greater than one. The term "combined molar amount" means the sum of the molar amount of NO and $NO_2$ that react according to reaction (2), above. Typically the molar ratio of ROH to (NO + $NO_2$) will be from greater than 1 to about 10, preferably from greater than 1 to about 2 and most preferably from greater than 1 to about 1.5.

In addition, it has been observed that by correlating the process temperature with the ratio of alcohol to the theoretical amount of $N_2O_3$ formed (based on NO and $NO_2$) the formation of acid by-products decreases or does not increase proportionately (i.e. the molar ratio of alkyl nitrite to acid by-product per unit of time increases). For example by correlating the temperature and alcohol to $N_2O_3$ ratio such that the temperature is between about 95°C and about 130°C, preferably above about 115°C and the alcohol to combined molar amount of nitric oxide and nitrogen dioxide is preferably about two-to-one the rate of formation of acid by-products can be minimized.

The process of this invention may be practiced in almost any commercial reactor and is generally carried out on a continuous basis by employment of two reaction zones comprising two separate reactors although a single reactor may be employed to contain the

primary and secondary reaction zones. The contact time (or residence time in the reactor) during which the gaseous materials react to form the esters of nitrous acid is generally between about 0.01 and about 50 seconds, preferably 0.1 to about 10 seconds for both the primary and secondary reaction zones. Shorter or longer times may be employed depending on the temperature, pressure, molar ratios, diluent and feed rate employed so long as sufficient time for reaction is provided. It has been observed that it may be preferably to employ a longer contact time in the secondary reaction zone such that the residence time in the secondary reaction zone due to the nature of the reaction occurring therein. In addition, the selection of the reactor geometry of the nitrite forming reaction zones will affect the actual residence time employed.

When the process is carried out in a continuous manner the feed rate is not narrowly critical, and is selected to satisfy the particular design of the continuous system.

The following description of the Experimental Procedure, Figure and the Examples are provided to illustrate this invention and are not intended to limit, in any way, the scope of this invention.

## EXPERIMENTAL PROCEDURE

The following examples were carried out using an apparatus as depicted in FIG. 1. The design of the apparatus is a particularly convenient design for carrying out the process of the invention although alternative designs may be employed. For the sake of simplicity, control valves, flow meters of common design and application and the like are not shown since they are of a design as are commonly available from commercial suppliers and their use is known to one skilled in the art.

In carrying out the examples aliphatic alcohol, ethanol or methanol, was introduced at line 10 and mixed with inert diluent in line 12 (shown as nitrogen) at point 14 of line 10 prior to introduction to reactor 26. Molecular oxygen is introduced at line 16 and a nitrogen oxide composition as introduced at line 18 with

admixture of the oxygen and the nitrogen oxide occurring at 20 with inert diluent (shown as nitrogen) being admixed at 25 prior to introduction to reactor 26. The construction of lines 10, 12, 16, 18 and 22 were of 6.35 mm (outside diameter) No. 304 stainless steel tubing having a wall thickness of 0.89 mm.

Reactor 26 comprises primary reaction zone 28, i.e., the upper section of the reactor, with the middle section 30 comprising an ethanol vaporization coil and lower section 32 comprising a preheater for the gaseous mixture of oxygen, nitrogen oxide and inert diluent. The primary reaction zone 28 comprised a 2.13 meter coiled section of 9.52 mm (outside diameter; 0.89 mm wall) No. 304 stainless steel tubing. The ethanol vaporization coil 30 comprised a 4.88 meter coiled section of a 12.7 mm (outside diameter) No. 304 stainless steel tubing; and the gas preheater coil 32 comprised a 6.10 meter coiled section of 9.52 mm (outside diameter; 0.89 mm wall) No. 304 stainless steel tubing. The outer shell 33 of reactor 26, wherein the upper, middle and lower sections are contained, comprises a 1.22 meter section of a 152 mm (outside diameter) mild steel.

The primary reaction zone 28 was connected to the secondary reaction zone 36 by 9.52 mm (outside diameter; wall thickness 0.89 mm) No. 304 stainless steel tubing. The secondary reaction zone 36 comprised a 0.30 meter, 203 mm section of 38.1 mm (outside diameter) No. 304 stainless steel jacketed by a 0.30 meter, 38.1 mm section of 63.5 mm (outside diameter) No. 304 stainless steel piping.

The secondary reaction zone 36 is connected to brine condenser 42 formed of a 0.61 meter, 152.4 mm section of 152.4 mm (outside diameter) No. 304 stainless steel pipe sheet containing 1.52 meter 254 mm of 12.7 mm (outside diameter) No. 304 stainless steel tubing. The brine condenser was connected to vapor-liquid separator 46 which was formed of a 0.30 meter, 152.4 mm section of 102 mm (outside diameter) No. 304 stainless steel pipe. The secondary reaction zone 36, condenser 42 and vapor-liquid vaporizer 46 were connected with 9.52 mm (outside diameter; 0.89 mm wall thickness) No. 304 stainless steel tubing (lines 40 and 44).

The apparatus will advantageously include such features as

detectors for CO, NO, alkyl nitrite and nitrogen oxides; pressure valves and regulators; temperature detectors; flow meters; and the like are of the type commercially available.

In carrying out the examples the primary reactor 26 and secondary reaction zone 36 were jacketed to provide for temperature control by cold water cooling or by THERMINOL™ or steam heating. The temperature was carefully monitored by standard temperature measurement devices. Reaction products were analyzed by vapor pressure chromatography.

EXAMPLES 1-13

The process according to this invention was carried out according to the above described experimental procedure employing nitric oxide (NO) as the nitrogen oxide and ethanol as the alcohol. The results, set forth in Table I, show that the rate of formation of acid by-products in the reaction product was less than 0.018 moles/hour total acid formed in most cases while the productivity and efficiency of ethyl nitrite remained high.

Table I

| Example | $NO/O_2$ [1] | EtOH [2] ($N_2O_3$) | Press [3] | Temp [4] | ETN [5] | $HNO_3$ [6] |
|---|---|---|---|---|---|---|
| 1 | 3.92 | 2.00 | 1.02 | 95.0 | 0.848 | 0.003 |
| 2 | 6.01 | 1.98 | 1.02 | 115.0 | 0.739 | 0.001 |
| 3 | 3.85 | 2.87 | 1.02 | 115.0 | 0.735 | 0.004 |
| 4 | 5.99 | 3.09 | 1.02 | 95.0 | 0.638 | 0.039 |
| 5 | 4.00 | 2.02 | 2.04 | 115.0 | 1.309 | 0.004 |
| 6 | 5.99 | 2.03 | 2.04 | 95.0 | 1.242 | 0.007 |
| 7 | 3.99 | 2.95 | 2.04 | 95.0 | 1.157 | 0.018 |
| 8 | 5.98 | 2.96 | 2.04 | 115.0 | 0.938 | 0.007 |
| 9 | 4.98 | 2.49 | 1.53 | 105.0 | 1.043 | 0.003 |
| 10 | 3.97 | 2.63 | 2.04 | 95.0 | 1.171 | 0.041 |
| 11 | 4.23 | 2.27 | 1.02 | 95.0 | 0.754 | 0.003 |
| 12 | 4.00 | 3.01 | 1.02 | 115.0 | 0.757 | 0.007 |
| 13 | 4.00 | 2.01 | 2.04 | 115.0 | 1.383 | 0.006 |

[1]molar ratio

[2]molar ratio of ethanol (EtOH) to dinitrogen trioxide ($2NO + 1/2O_2 \rightleftharpoons N_2O_3$; based on theoretical yield of $N_2O_3$ from NO and $O_2$.

[3]gauge pressure in Pa x $10^5$.

[4]temperature in °C.

[5]ethyl nitrite, reported as moles per hour.

[6]total acid, reported s moles of $HNO_3$ per hour.

EXAMPLE 14

The process was carried out as in examples 1-13 except that the alcohol was methanol. The result is set forth in Table II.

Table 2

| Example | $NO/O_2$[1] | MeOH[2] ($N_2O_3$) | Press[3] | Temp[4] | MN[5] | $HNO_3$[6] |
|---|---|---|---|---|---|---|
| 14 | 5.99 | 1.93 | 1.02 | 115.0 | 0.743 | Trace |

---

[1]molar ratio

[2]molar ratio of methanol to dinitrogen trioxide ($2NO + 1/2O_2 \rightleftharpoons N_2O_3$ based on theoretical yield of $N_2O_3$ from NO and $O_2$.

[3]gauge pressure in Pa x $10^5$.

[4]temperature in °C.

[5]methyl nitrite; reported as moles formed per hour.

[6]total acid reported as moles of $HNO_3$ per hour.

## CLAIMS

1. The process for the preparation of esters of nitrous acid in the vapor state comprising the steps of

(a) introducing a nitrogen oxide composition, alcohol and inert diluent into a first reaction zone wherein is optimized the reaction

$$N_2O_3 + ROH \longrightarrow RONO + HONO$$

at a temperature pressure and for a time sufficient to form said ester of nitrous acid;

(b) introducing the mixture of step (a) to a second reaction zone wherein is optimized the reaction

$$ROH + HONO \longrightarrow RONO + H_2O$$

at a temperature, pressure and for a time sufficient to form said ester of nitrous acid; and

(c) collecting the ester of nitrous acid from step (b) wherein the formation of acid by-products is minimized.

2. The process of claim 1 wherein said second reaction zone is packed.

3. The process of claim 2 wherein the inert diluent is nitrogen.

4. The process of claim 1 wherein the temperature in said first and second reaction zones is between about 50 and about 140°C.

5. The process of claim 4 wherein the temperature is between about 95 and about 120°C.

6. The process of claim 1 wherein the pressure is atmospheric or superatmospheric pressure.

7. The process of claim 1 wherein the time is between about

0.01 seconds and about 50 seconds.

8. The process of claim 1 wherein said nitrogen oxide composition contains nitric oxide and nitrogen dioxide in a molar ratio of greater than 4 to 1 and the molar ratio of aliphatic alcohol to combined molar amount of nitric oxide and nitrogen dioxide is greater than one.

9. The process of claim 1 wherein said process is carried out under essentially anhydrous conditions.

10. The process of claim 8 wherein said temperature is between about 95°C and 130°C and preferably between 115 and 130°C and is correlated with said molar ratio of alcohol to combined molar amount of nitric oxide to provide for a minimum formation of acid by-products.

0076217